# EUROPÄISCHE PATENTSCHRIFT

(11) **EP 3 742 993 B1**
(45) Veröffentlichungstag und Bekanntmachung des Hinweises auf die Patenterteilung: **23.03.2022**
(21) Anmeldenummer: 19711241.0
(22) Anmeldetag: 23.01.2019
(51) Int. Cl.: A61B 17/70, A61B 17/86

(54) **INTRAVERTEBRALE SCHRAUBE**
INTERVERTEBRAL SCREW
VIS INTRAVERTÉBRALE

(30) Priorität: 23.01.2018 DE 202018100353 U
(43) Veröffentlichungstag der Anmeldung: 02.12.2020
(73) Patentinhaber: Rosenthal, Daniel, 60598 Frankfurt am Main (DE)
(72) Erfinder: Rosenthal, Daniel, 60598 Frankfurt am Main (DE)
(74) Vertreter: LS-MP von Puttkamer Berngruber Loth Spuhler
(86) Internationale Anmeldenummer: PCT/DE2019/100068
(87) Internationale Veröffentlichungsnummer: WO 2019/144996

(56) Entgegenhaltungen:
- DE-A1- 19 913 770
- US-A1- 2011 230 920
- US-A1- 2012 010 668
- US-A1- 2017 231 715

## Beschreibung

Die Erfindung betrifft eine chirurgische Schraube zum Einführen in den Knochen eines menschlichen oder tierischen Körpers nach dem Oberbegriff des Anspruchs 1.

Aus der CH 703 411 A2 ist eine Pedikelschraube bekannt, die einen Schaft umfasst, an dessen Außenseite ein Gewinde angeordnet ist. An dem hinteren Ende der Schraube ist ein Kopf vorgesehen. An den Kopf wird ein Werkzeug angelegt, um die Pedikelschraube in eine Rotation um ihre Längsachse zu versetzen. Durch die Rotation wird die Pedikelschraube in den Knochen eines menschlichen und/oder tierischen Körpers eingeschraubt.

Bekannte chirurgische Schrauben zum Einführen in einen Knochen erweisen sich als nachteilhaft, da der Kopf der Schraube auf dem Schaft der Schraube sitzt. Hierbei grenzt der große Durchmesser des Kopfes an den relativ zum Kopf kleineren Durchmesser des Schaftes der chirurgischen Schraube. Der Kopf der chirurgischen Schraube kann bei zunehmender Belastung vom Schaft abbrechen.

Andere bekannte chirurgische Schrauben ragen nach dem Einschrauben in den menschlichen Knochen mit dem gesamten Kopf aus dem Knochen heraus. Es besteht dabei die Gefahr, dass der Kopf der Schraube im Körper angrenzende Organe reizt oder verletzt.

Der aus dem Knochen herausragende Kopf der Schraube ragt nach erfolgreich durchgeführter Operation für den Patienten fühlbar aus dem Knochen heraus.

Dies erweist sich insbesondere dann als nachteilig, wenn die Schraube in Bezug auf den menschlichen Körper gesehen von vorne oder seitlich in den Knochen eingeschraubt wird.

Wenn der Kopf der Schraube in vollem Umfang aus dem Knochen herausragt, liegt der Schwerpunkt der Schraube relativ weit am Außenumfang des menschlichen Körpers. Hierdurch kann es bei zunehmender Belastung der Schraube zu Beschädigungen des Knochens kommen.

Eine chirurgische Schraube mit den Merkmalen des Oberbegriffs des Anspruchs 1 ist aus der US 2011/230920 A1 bekannt.

Die Erfindung wird gelöst durch die Merkmale des Anspruchs 1.

Es ist eine chirurgische Schraube vorgesehen, der in den Knochen eines menschlichen oder tierischen Körpers eingeführt wird.

Die Erfindung betrifft eine chirurgische Schraube, vorzugsweise eine modifizierte intravertebrale Schraube. Die chirurgische Schraube kann auch ein Stift sein.

Im Weiteren wird davon ausgegangen, dass die chirurgische Schraube eine intravertebrale Schraube ist.

### Chirurgische Schraube in Form einer intravertebralen Schraube

Die intravertebrale Schraube bezeichnet ein chirurgisches Instrument zur Stabilisierung der Wirbelsäule in Form einer Schraube. Es versteht sich jedoch von selbst, dass die chirurgische Schraube, insbesondere die intravertebrale Schraube auch in jeden anderen Knochen des menschlichen oder tierischen Skeletts eingeführt werden kann.

Die intravertebrale Schraube kann als Kortikalisschraube ausgebildet sein. Die Kortikalisschraube dient der operativen Frakturbehandlung. Zur Fixierung in dem Knochen weist die Kortikalisschraube ein Außengewinde mit Profiltälern und Profilkämmen auf.

Das Anziehen der Kortikalisschraube bewirkt bei der Operation einen kontinuierlichen Druck. Durch diesen Druck wird eine Vorspannung erreicht, die dem Knochenbau weitere Stabilität zuführt.

Mit der Erfindung wird eine größere Kortikalis-Verankerung erreicht. Die intravertebrale Schraube weist einen Schaft auf. Am einen Enden des Schaftes umfasst die intravertebrale Schraube eine konisch verlaufende Spitze zum leichteren Einführen der intravertebralen Schraube in den Knochen.

Am gegenüberliegenden Ende der intravertebralen Schraube ist ein Kopf vorgesehen.

Der Kopf der intravertebralen Schraube weist Formelemente auf, die zur Ausführung einer radialen Bewegung durch die intravertebrale Schraube mit einem Werkzeug in Kontakt bringbar sind.

Die Schraube weist zumindest bereichsweise ein Außengewinde auf.

Das Außengewinde ist an wenigstens einem Abschnitt des Schaftes der intravertebralen Schraube radial am Außenumfang des Schaftes angeordnet.

Das Außengewinde der intravertebralen Schraube ist an wenigstens einem Abschnitt des Kopfes radial am Außenumfang des Kopfes angeordnet.

Die intravertebrale Schraube kann dauerhaft im Knochen verankert werden. Alternativ wird die intravertebrale Schraube nach einer vorbestimmten Zeitdauer wieder aus dem Knochen entfernt.

An ihrem der Spitze gegenüberliegenden freien Ende kann die intravertebrale Schraube mit einem chirurgischen Stabilisierungselement verbunden sein.

Das chirurgische Stabilisierungselement kann außerhalb des Körpers oder im inneren des menschlichen Körpers angeordnet sein.

Die intravertebrale Schraube weist vorzugsweise, aber nicht ausschließlich eine zylindrische Form auf.

Die intravertebrale Schraube weist ein Außengewinde auf, das in den Knochen eingreift.

Die intravertebrale Schraube wird kraft- und/oder formschlüssig im Knochen verankert. Die Verankerung erfolgt vorzugsweise mittels eines Außengewindes. Es kann jedoch auch jede andere Verankerungsform im Knochen gewählt werden. Die Verbindung ist lösbar.

Die intravertebrale Schraube ist aus einem nicht rostenden Stahl gefertigt. Die intravertebrale Schraube kann auch aus einer Metalllegierung oder aus Keramik oder aus Kunststoff gefertigt sein.

Zur Fertigung der intravertebralen Schraube kann jedes Material herangezogen werden, das vom Körper vertragen wird und insbesondere keine allergischen und/oder Abstoßungsreaktionen hervorruft. Beispielhaft und nicht ausschließlich ist die intravertebrale Schraube aus einem Knochen gefertigt. Sie kann auch aus Karbon oder jedem anderen Werkstoff hergestellt sein.

Die intravertebrale Schraube ist im Knochen verankert. Alternativ kann die intravertebrale Schraube den Knochen durchdringen. Zu beiden Seiten des Knochens kann ein chirurgisches Stabilisierungselement angeordnet werden.

Die intravertebrale Schraube nimmt insbesondere Querbelastungen auf.

Die intravertebrale Schraube weist über die gesamte Schraubenlänge einen einheitlichen Durchmesser auf. Alternativ kann die Schraube über die Länge des Schaftes unterschiedliche Schaftdurchmesser haben.

Die Anordnung der intravertebralen Schraube erweist sich als vorteilhaft, da sie je nach Beschaffenheit des Knochens eine unterschiedliche Länge und/oder einen unterschiedlichen Durchmesser annehmen kann.

### Kopf und Hals der intravertebralen Schraube

Der Kopf der intravertebralen Schraube weist Formelemente auf.

Die Formelemente dienen zum Eingriff von Werkzeugen. Der Eingriff des jeweiligen Werkzeugs in das Formelement des Kopfes dient dazu, die intravertebrale Schraube in eine Rotation um ihre Längsachse zu versetzen.

Durch die Rotation um ihre Längsachse wird die intravertebrale Schraube in den Knochen eingeschraubt.

Der Kopf der intravertebralen Schraube kann aus demselben Material gefertigt sein wie der Schaft der intravertebralen Schraube. Es versteht sich von selbst, dass der Kopf der intravertebralen Schraube auch aus einem anderen Material hergestellt werden kann. Der Kopf der intravertebralen Schraube kann als Flachkopf, als Tellerkopf, als Rundkopf, als Sechskantkopf oder als Senkkopf, als Senkrundkopf, als Zylinderkopf, als Flachrundkopf, als Panhead oder als Senkfräskopf ausgebildet sein.

Das Werkzeug kann auch ein Schraubenzieher sein.

Zur kraftschlüssigen und/oder formschlüssigen Anordnung des Werkzeugs am Kopf der intravertebralen Schraube können die Formelemente am Außenumfang des Kopfes der intravertebralen Schraube angeordnet sein.

Die Formelemente des Kopfes können auch auf dem von der Spitze der intravertebralen Schraube abgewandten Ende des Kopfes angeordnet sein.

Der Kopf der intravertebralen Schraube kann einen Innen-Vierkantantrieb, einen Innen-Sechskantantrieb oder einen Innen-Vielzahnantrieb aufweisen.

Zum Einführen der intravertebralen Schraube in den Knochen kann auch jeder andere Schraubenkopfantrieb gewählt werden.

Erfindungsgemäß weist der Kopf der intravertebralen Schraube zumindest an wenigstens einem Abschnitt des Kopfes ein Außengewinde auf, das durch die Erstreckung des Außengewindes des Schaftes auf diesem Abschnitt gebildet wird.

Hierdurch wird erreicht, dass die intravertebrale Schraube beim Einführen in den Knochen sowohl mit ihrem Schaft als auch mit dem Abschnitt des Kopfes in den Knochen eindringt, der das Außengewinde aufweist.

Der Kopf der intravertebralen Schraube weist eine Bohrung auf, die der Aufnahme von orthopädischen Stabilisierungselementen dient, die vorzugsweise rechtwinklig zum Schaft der intravertebralen Schraube verlaufen.

Auf diese Weise können wenigstens zwei, vorzugsweise parallel zueinander angeordnete intravertebrale Schrauben miteinander verbunden werden.

Das orthopädische Stabilisierungselement zur Verbindung von wenigstens zwei intravertebralen Schrauben kann stabförmig ausgebildet sein.

Das Stabilisierungselement verbindet wenigstens zwei Knochen des Skeletts miteinander. Es kann zur Verbindung wenigstens zweier Bereiche eines einzelnen Knochens eingesetzt werden.

Das Stabilisierungselement kann ein Stab oder eine Platte sein. Alternativ kann das Stabilisierungselement ein Seil sein. Es versteht sich von selbst, dass das orthopädische Stabilisierungselement auch ein anderer Gegenstand sein kann oder eine andere geometrische Form aufweisen kann.

Das Stabilisierungselement kann ein elastischer oder ein nicht elastischer Körper sein. Das Stabilisierungselement kann nach erfolgter Stabilisierung der Knochen im Körper verbleiben. Alternativ wird das Stabilisierungselement nach der Operation wieder aus dem Körper entfernt.

Zur Fixierung des orthopädischen Stabilisierungselements im und/oder am Kopf sind vorzugsweise hexagonale Schrauben vorgesehen. Zur Befestigung des orthopädischen Stabilisierungselements kann alternativ eine in den Kopf eingebettete Mutter vorgesehen sein. Das orthopädische Stabilisierungselement kann seitlich in die Bohrung des Kopfes eingeführt sein. Zur Fixierung des orthopädischen Stabilisierungselements am Kopf ist eine senkrecht verlaufende Schraube vorgesehen.

Alternativ können auch andere Mittel zur Fixierung des orthopädischen Stabilisierungselements im Kopf vorgesehen sein.

Zwischen dem Schaft der intravertebralen Schraube und dem Kopf der intravertebralen Schraube kann ein Hals angeordnet sein.

Zumindest an einem Abschnitt des Halses ist das Außengewinde radial am Hals angeordnet.

Das am Schaft angeordnete Außengewinde geht in das am Hals angeordnete Außengewinde über.

Das am Hals angeordnete Außengewinde der intravertebralen Schraube geht in das Außengewinde des Kopfes der intravertebralen Schraube über.

Der Hals kann denselben Durchmesser aufweisen wie der Schaft der intravertebralen Schraube.

Der Hals kann einen Durchmesser aufweisen, der dem Durchmesser des Kopfes der intravertebralen Schraube entspricht. Alternativ kann der Hals der intravertebralen Schraube zur Verbindung des Schaftes mit dem Kopf einen sich in Richtung des Kopfes hin aufweitenden Durchmesser aufweisen.

Der Schaft und der Hals sowie der Kopf des Halses weisen voneinander abweichende Durchmesser auf.

Alternativ können der Schaft und der Hals und der Kopf der chirurgischen Schraube, insbesondere der intravertebralen Schraube in Bezug auf ihren Durchmesser übereinstimmen.

Der Durchmesser kann über die Länge des Kopfes der intravertebralen Schraube und/oder über die Länge des Halses der intravertebralen Schraube variieren.

Eine derartige Konstruktionsweise hat den Vorteil, dass die intravertebrale Schraube besonders gut im Knochen verankert ist.

Es ist vorgesehen, dass der Kopf der intravertebralen Schraube am Schaft angelenkt ist.

Der Kopf kann auch auf eine andere technische Art und Weise am Schaft der intravertebralen Schraube angebracht sein. Die Erfindung erweist sich als vorteilhaft, da die intravertebrale Schraube auf diese Weise von vorne, von der Seite oder von Hinten, vorzugsweise in einen Wirbel der Wirbelsäule des Patienten eingeschraubt werden kann.

An wenigstens einem Abschnitt ist am Kopf der intravertebralen Schraube ein Außengewinde angeordnet.

Konstruktionsbedingt dringt neben dem Schaft der intravertebralen Schraube auch der mit dem Außengewinde versehene Abschnitt des Kopfes der intravertebrale Schraube in den Knochen ein.

Zusätzlich dringt der zwischen dem Schaft und dem Kopf angeordnete Hals in den Knochen ein.

Bei Verbleib der intravertebralen Schraube im Knochen eines Patienten kann somit eine intravertebrale Schraube ausgewählt werden, die einen relativ gesehen kürzeren Schaft aufweist.

Ein weiterer Vorteil besteht darin, dass die intravertebrale Schraube nur in einem geringen Umfang mit ihrem Kopf aus dem Knochen herausragt.

Nur der Abschnitt des Kopfes der intravertebralen Schraube ragt aus dem Knochen heraus, der nicht mit einem Außengewinde versehen ist.

Der Schaft der intravertebralen Schraube kann in dem axialen Umfang kürzer sein, in dem der Kopf der intravertebralen Schraube mit einem Außengewinde versehen ist.

Der Kopf der intravertebralen Schraube wird in dem Umfang, in dem am Kopf ein Außengewinde angeordnet ist zusätzlich zum Schaft der intravertebralen Schraube in den Knochen eingeführt.

Es erweist sich als vorteilhaft, dass die intravertebrale Schraube in Röhrenknochen und in platte Knochen, in kurze Knochen, in Sesambeine, sowie in luftgefüllte Knochen und in unregelmäßige Knochen eingeschraubt werden kann.

Aufgrund des Halses ist das Außengewinde des Schaftes der intravertebralen Schraube in das Außengewinde des Kopfes der intravertebralen Schraube überführbar und/oder verlängerbar.

Der Schraubenkopf und/oder der Hals der intravertebralen Schraube wird zum funktionalen Bestandteil des Außengewindes. Erfindungsgemäß wird eine verbesserte Verankerung der intravertebralen Schraube im Knochen erreicht.

Konstruktionsgemäß kann die intravertebrale Schraube von der Spitze der intravertebralen Schraube bis zu ihrem Kopf hin eine Trichterform aufweisen.

Die Trichterform ermöglicht eine besonders feste und gleichmäßige Verankerung der intravertebralen Schraube im Knochen.

Ein weiterer Vorteil der Erfindung besteht darin, dass Schäfte, Hälse und Köpfe unterschiedlicher Gestalt und Ausprägungsform bei der Herstellung der miteinander kombiniert werden können.

Aufgrund des mit seinem Außengewinde in den Knochen hineinragenden Kopfes verringert sich das Profil der intravertebralen Schraube, mit dem die intravertebrale Schraube aus dem Knochen herausragt.

### Außengewinde

Das Außengewinde ist wenigstens an einem Abschnitt am Schaft der intravertebralen Schraube und erstreckt sich auf wenigstens einem Abschnitt des Kopfes der Schraube Vorzugsweise ist zwischen dem Schaft und dem Kopf der intravertebralen Schraube an dessen Hals ein Außengewinde angeordnet.

Unter dem Begriff Außengewinde eine profilierte Einkerbung zu verstehen, die sich in Form einer Wendel kontinuierlich oder zumindest abschnittsweise um den Außenumfang des Schaftes und um den Außenumfang des Halses und um den Außenumfang des Kopfes der intravertebralen Schraube erstreckt. Die profilierte Einkerbung umfasst einen Profilkamm. Der Profilkamm verläuft zwischen zwei Profiltälern.

Das Außengewinde ist als Rechtsgewinde ausgebildet. Alternativ kann das Außengewinde jeweils auch als Linksgewinde gefertigt sein.

Das Außengewinde am Schaft und/oder am Hals und/oder am Kopf der intravertebralen Schraube ist jeweils als selbstschneidendes Gewinde ausgebildet. Es versteht sich von selbst, dass die Gewindegänge am Schaft, am Hals und am Kopf der intravertebralen Schraube auch eine andere Gewindeform aufweisen können.

Der Hals der intravertebralen Schraube weist erfindungsgemäß ein Außengewinde auf. Alternativ kann der Hals der intravertebralen Schraube auch zwischen zwei Gewindegängen angeordnet sein.

Der Hals der intravertebralen Schraube kann denselben Durchmesser aufweisen, wie der Schaft der intravertebralen Schraube.

Alternativ kann der Hals der intravertebralen Schraube einen sich vom Schaft zum Kopf hin weitenden Durchmesser aufweisen.

Rein beispielhaft und in keiner Weise ausschließlich kann der Durchmesser des Schaftes im Verhältnis zur kumulierten axialen Länge des Schaftes und des Abschnittes des Kopfes, der mit einem Außengewinde versehen ist 4,5 mm x 20 mm oder 6,5 mm x 20 mm oder 7,5 mm x 20 mm betragen.

Ebenfalls rein beispielhaft und in keiner Weise ausschließlich kann der Durchmesser des Außengewindes am Kopf der intravertebralen Schraube 12,5 mm betragen.

In einem weiteren Beispiel beträgt die axiale Läge des mit einem Außengewinde versehenen Abschnittes des Kopfes der intravertebralen Schraube 6,35 mm.

Der übrige Abschnitt des Kopfes, der kein Außengewinde aufweist, kann rein beispielhaft eine Länge von 12 mm aufweisen.

In einem weiteren Beispiel beträgt der Abstand zwischen zwei Gewindegängen im Bereich des Halses der intravertebralen Schraube 4,6 mm.

In einem anderen Ausführungsbeispiel kann der Abstand zwischen zwei Gewindegängen im Bereich des Halses der intravertebralen Schraube 10,2 mm betragen.

Sämtliche vorausgehend genannten Durchmesser- und Längenangaben sind lediglich beispielhaft gemeint und können auch andere Werte annehmen.

### Kanal

Es ist ein Kanal angeordnet, der sich entlang der Längsachse der intravertebralen Schraube axial durch den Schaft der intravertebralen Schraube erstreckt. Der Kanal verläuft durch den Hals und/oder durch den Kopf der intravertebralen Schraube. Entlang der Längsachse der intravertebralen Schraube erstreckt sich der Kanal vom Kopf der intravertebralen Schraube bis zu deren Spitze.

Der Kanal ist zur Aufnahme wenigstens eines Gegenstands ausgebildet.

Entlang der Längsachse der intravertebralen Schraube ist ein Draht oder eine Innenschraube in den Kanal einführbar. Die Innenschraube ist eine in die intravertebrale Schraube einführbare Schraube, mit relativ zur intravertebralen Schraube kleinerem Durchmesser. Es versteht sich von selbst, dass auch ein anderer Gegenstand z.B. eine Kanüle in die intravertebrale Schraube eingeführt werden kann.

Der in den Kanal im Inneren des Schaftes und/oder des Kopfes und/oder des Halses der intravertebralen Schraube einführbare Gegenstand ist eine Innenschraube oder ein Bolzen oder ein Stift.

Der Gegenstand wird in den Kanal eingeschraubt. Der Gegenstand kann hineingeschoben oder in den Kanal gezogen werden.

Alternativ kann der Gegenstand mit einer Zugeinrichtung in den Kanal der intravertebralen Schraube hinein gezogen werden. Die Zugeinrichtung kann beispielhaft und in keiner Weise ausschließlich ein Faden oder eine Kette sein.

Der Gegenstand kann einen kleineren Durchmesser aufweisen, als der Kanal.

Der Kanal dient als Zieleinrichtung oder Leitung für eine präzise Platzierung der intravertebralen Schraube im Knochen.

Weiter ist eine Spreizeinrichtung angeordnet. Hierbei weist der Gegenstand, der in die intravertebralen Schraube eingeführt wird, einen größeren Durchmesser auf, als der Kanal im Inneren der intravertebralen Schraube.

Beispielhaft und nicht ausschließlich hat der Kanal einen Durchmesser von 2 bis 3 mm. Der Durchmesser kann auch einen anderen Wert annehmen.

Durch Einführen des Gegenstands mit dem größeren Durchmesser in den Kanal werden der Schaft und/oder der Hals und/oder der Kopf der intravertebralen Schraube aufgeweitet.

Durch Einführen des Gegenstands mit dem größeren Durchmesser in den Kanal kann der Schaft und/oder der Hals und/oder der Kopf der intravertebralen Schraube verformt werden.

Alternativ können der Schaft und/oder der Hals und/oder der Kopf der intravertebralen Schraube die bisherige Form beibehalten, so dass lediglich das Außengewinde des Schaftes und/oder des Halses und/oder des Kopfes verformt werden.

Der in die intravertebrale Schraube eingeführte Gegenstand wird zumindest bereichsweises wieder aus der intravertebralen Schraube herausgezogen. Dabei wird die Spitze der intravertebralen Schraube zumindest stückweise mitgezogen. Der Abschnitt des Schaftes wird auf diese Weise gegenüber dem umgebenden Knochen dicker und runder.

Hierdurch gewinnt der Schaft zusätzlich an Fläche. Der Kopf bildet eine Gegenverankerung zum Kopf der intravertebralen Schraube zum umgebenden Knochen.

Die intravertebralen Schraube spreizt sich nach ihrer Aufweitung ähnlich eines Dübels radial in den Knochen hinein.

Durch die Aufweitung der intravertebralen Schraube (3) kann die Verankerung der Schraube im Knochen zusätzlich verbessert werden.

### Kenngrößen

Bei den Kenngrößen des Außengewindes handelt es sich um den Flankendurchmesser oder um den Kerndurchmesser oder um den Steigungswinkel des Gewindes.

Als Kerngröße des Außengewindes bezeichnet die Erfindung auch die Mehrgängigkeit bzw. die Eingängigkeit des Außengewindes.

Der Flankendurchmesser bezeichnet dabei denjenigen Durchmesser, eines gedachten geometrischen Kreiszylinders, der das Gewindeprofil in der Weise durchschneidet, dass die Breiten der jeweils entstehenden Profiltäler und Profilkämme gleich groß sind.

Der Kerndurchmesser des Außengewindes bezeichnet den kleinsten Durchmesser der Gewindegeometrie.

Mit dem Begriff Steigungswinkel ist der Winkel gemeint, den die Flanke des Außengewindes zu einer Längsachse der intravertebralen Schraube einnimmt.

Erfindungsgemäß bezeichnet eine weitere Kenngröße des Außengewindes die Mehrgängigkeit bzw. die Eingängigkeit des Außengewindes.

Das eingängige Außengewinde verläuft in Form einer profilierten Einkerbung wendelartig, um die Wandung der intravertebralen Schraube.

Bei einem mehrgängigen Außengewinde verlaufen mehrere Gewindegänge parallel zueinander.

Die Kenngrößen des am Schaft angeordneten Außengewindes und des am Hals angeordneten Außengewindes, sowie des am Kopf angeordneten Außengewindes sind gleich.

Alternativ können sich die jeweiligen Kenngrößen des am Schaft angeordneten Außengewindes und des am Hals angeordneten Außengewindes, sowie des am Kopf angeordneten Außengewindes unterscheiden.

Weitere Vorteile und Ausgestaltungen der Erfindung sind aus der nachfolgenden Zeichnung zu ersehen. Hierbei zeigen:
Fig. 1 zeigt zwei Wirbel eines menschlichen Skeletts mit intravertebralen Schrauben,
Fig. 2 zeigt einen Teil einer intravertebralen Schraube mit einem Schaft, einem Hals und einem Kopf und
Fig. 3 zeigt eine intravertebrale Schraube mit Hals, der ein Außengewinde aufweist.

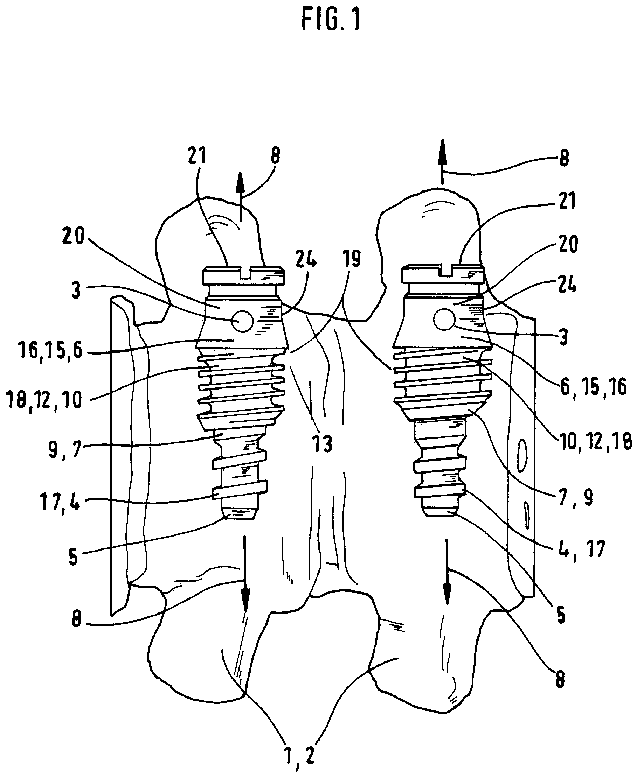

Die Fig. 1 zeigt zwei aneinander anstoßende Knochen 1 in Form von Wirbeln 2 eines menschlichen Skeletts.

Seitlich in jedem Wirbel 2 ist je eine intravertebrale Schraube 3 eingeschraubt. Die intravertebralen Schrauben 3 sind in etwa rechtwinklig zu der Wirbelsäule des Skeletts angeordnet.

In der Fig. 1 weist die intravertebrale Schraube 3 einen Schaft 4 auf. Zum Eindringen des Schaftes 4 in den Wirbel 2 umfasst die intravertebrale Schraube 3 eine Spitze 5.

An dem von der Spitze 5 abgewandten Ende der intravertebralen Schraube 3 ist ein Kopf 6 angeordnet.

Zwischen dem Schaft 4 und dem Kopf 6 weist die intravertebrale Schraube 3 einen Hals 10 auf.

Entlang einer Längsachse 8 der intravertebralen Schraube 3 ist an dem Schaft 4 ein Außengewinde 7 angeordnet. Das Außengewinde 7 erstreckt sich in einem Abschnitt 9 des Schaftes 4.

Je ein Außengewinde 7 ist jeweils an einem Abschnitt 12 am Hals 10 angeordnet.

Ein Abschnitt 13 des Kopfes 6 weist am Außenumfang 15 des Kopfes 6 ein Außengewinde 16 auf.

Das Außengewinde 17 geht in das Außengewinde 18 am Hals 10 über.

Das Außengewinde 18 des Halses 10 geht nach der Darstellung der Fig. 1 in das Außengewinde 16 des Kopfes 6 über.

Das Außengewinde 17 des Schaftes 4 und das Außengewinde 18 des Halses 10 sowie das Außengewinde 16 des Kopfes 6 weisen jeweils unterschiedliche Durchmesser auf.

Mit einem Abschnitt 19 ist der Kopf 6 im Bereich seines Außengewindes 16 im Wirbel 2 verankert.

Ein Abschnitt 20 des Kopfes 6 weist kein Außengewinde 16 auf.

Mit dem Abschnitt 20 ragt der Kopf 6 aus dem Wirbel 2 heraus.

An dem von der Spitze 5 abgewandten Ende des Kopfes 6 weist der Kopf 6 ein Formelement 21 auf.

Ein (nicht dargestelltes) Werkzeug greift in das Formelement 21 des Kopfes 6 ein.

Mithilfe des Werkzeuges wird die intravertebrale Schraube 3 in eine Rotation um ihre Längsachse 8 versetzt.

Durch die Rotation wird die intravertebrale Schraube 3 in den Knochen 1, im Fall der Fig. 1 den Wirbel 2 eingeschraubt.

In etwa achsparallel zur Wirbelsäule weisen die Köpfe 6 der intravertebralen Schrauben 3 je eine Bohrung 24 auf.

Die in der Fig. 1 gezeigten Bohrungen 24 fluchten miteinander.

Zur Verbindung der beiden in Fig. 1 gezeigten intravertebralen Schrauben 3 wird ein (nicht gezeigter) Stab achsparallel zur Wirbelsäule durch die Bohrungen 24 der Köpfe 6 der intravertebralen Schrauben 3 hindurchgeführt.

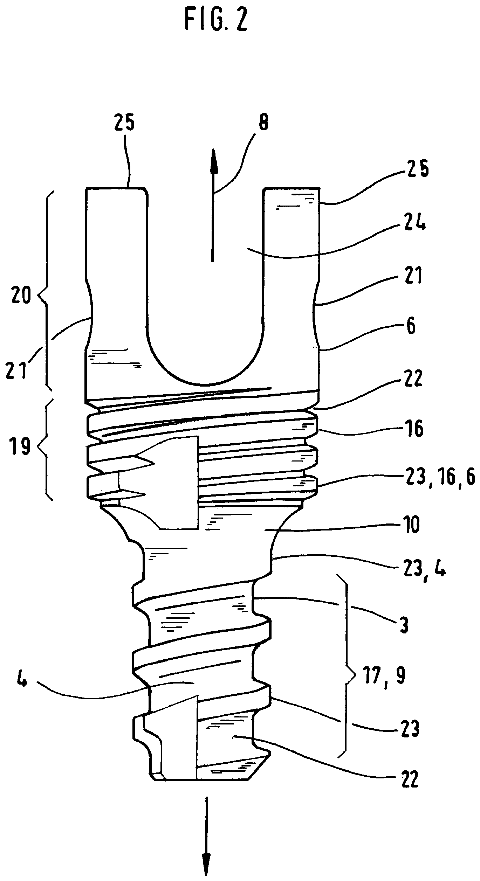

Die Fig. 2 zeigt eine intravertebrale Schraube 3. Die intravertebrale Schraube 3 umfasst den Schaft 4 und den Kopf 6.

Zwischen dem Schaft 4 und dem Kopf 6 ist in der Fig. 2 ein Hals 10 dargestellt.

In etwa rechtwinklig zur Längsachse 8 der intravertebralen Schraube 3 weist der Kopf 6 eine nach oben hin offene Bohrung 24 auf.

In etwa rechtwinklig zur Längsachse 8 der intravertebralen Schraube 3 wird in die Bohrung 24 ein (nicht gezeigter) Stab eingeführt.

Zur Bildung der Bohrung 24 weist der Kopf 6 eine durch wenigstens zwei Außenwände 25 gebildete U-förmige Aussparung auf. Die Aussparung dient zur Aufnahme des Stabs.

In dem Abschnitt 9 ist am Schaft 4 das Außengewinde 17 angeordnet.

Das Außengewinde 17 des Schaftes 4 weist Profiltäler 22 und Profilkämme 23 auf.

Der Hals 10 der intravertebralen Schraube 3 ist in der Fig. 2 zwischen zwei Profilkämmen 23 des Außengewindes 17 des Schaftes 4 und des Außengewindes 16 des Kopfes 6 angeordnet.

Der Abschnitt 19 des Kopfes 6 ragt mit seinem Außengewinde 16 in den (nicht dargestellten) Knochen 1 hinein.

Der Abschnitt 20 des Kopfes 6 der intravertebralen Schraube 3 ragt nach Einschrauben der intravertebralen Schraube 3 in den (nicht gezeigten) Knochen 1 aus diesem heraus.

In dem Außengewinde 16 des Kopfes 6 sind in der Fig. 2 die Profiltäler 22 sowie die Profilkämme 23 dargestellt.

Am Kopf 6 sind Formelemente 21 dargestellt. Die Formelemente 21 dienen zum Angriff von Werkzeugen, mit denen die intravertebrale Schraube 3 in den (nicht dargestellten) Knochen eingeschraubt wird.

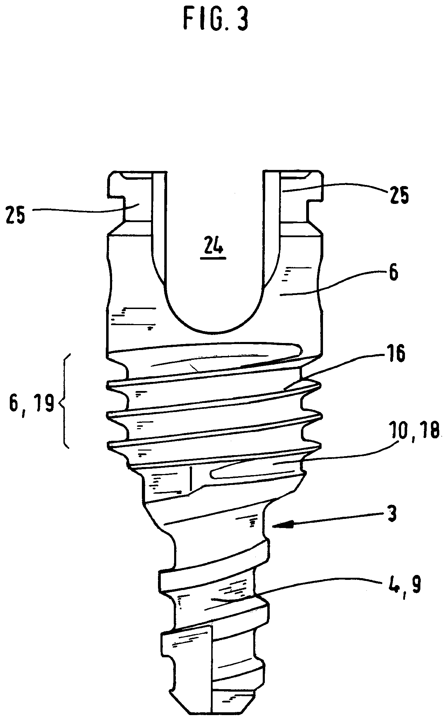

Fig. 3 zeigt eine intravertebrale Schraube 3 entsprechend der Fig. 2, mit dem Unterschied, dass der Hals 10 der intravertebralen Schraube 3 zwischen dem Schaft 4 und dem Kopf 6 ein Außengewinde 18 aufweist.

Nach Einschrauben der intravertebralen Schraube 3 in den (nicht gezeigten) Knochen ragt die intravertebrale Schraube 3 mit ihrem Schaft 4 und mit dem Hals 10 in den Knochen hinein.

Die in der Fig. 3 dargestellte intravertebrale Schraube 3 ragt nach Einschrauben in den Knochen weiter mit dem Abschnitt 19 des Kopfes 6 in den (nicht dargestellten) Knochen hinein, der das Außengewinde 16 aufweist.

Zur Bildung der Bohrung 24 weist der Kopf 6 in Fig. 3 eine durch wenigstens zwei Außenwände 25 gebildete U-förmige Aussparung auf. Die Aussparung dient zur Aufnahme des Stabs.

### Bezugsziffern

- 1: Knochen
- 2: Wirbel
- 3: intravertebrale Schraube
- 4: Schaft
- 5: Spitze
- 6: Kopf
- 7: Außengewinde
- 8: Längsachse
- 9: Abschnitt Schaft
- 10: Hals
- 11: freibleibend
- 12: Abschnitt Hals
- 13: Abschnitt Kopf
- 14: freibleibend
- 15: Außenumfang Kopf
- 16: Außengewinde Kopf
- 17: Außengewinde Schaft
- 18: Außengewinde Hals
- 19: Abschnitt des Kopfes im Knochen
- 20: Abschnitt des Kopfes, der aus dem Knochen herausragt
- 21: Formelement
- 22: Profiltal
- 23: Profilkamm
- 24: Bohrung im Kopf
- 25: Außenwand

## Patentansprüche

1. Chirurgische Schraube zur Fixierung in einem Knochen (1) eines menschlichen oder tierischen Körpers mit einem Kopf (6), mit einem Schaft (4) und mit einem Außengewinde (17), das an wenigstens einem Abschnitt (9) des Schaftes (4) der chirurgischen Schraube angeordnet ist, wobei sich das Außengewinde (16) auf wenigstens einem Abschnitt (13) des Kopfes (6) erstreckt, **dadurch gekennzeichnet, dass** der Kopf (6) eine durch wenigstens zwei Außenwände (25) gebildete U-förmige Aussparung zur Aufnahme eines Stabilisierungselements aufweist.

2. Chirurgische Schraube nach Anspruch 1, **dadurch gekennzeichnet, dass** die chirurgische Schraube eine intravertebrale Schraube (3) ist.

3. Chirurgische Schraube nach einem der Ansprüche 1 oder 2, **dadurch gekennzeichnet, dass** die chirurgische Schraube zwischen dem Schaft (4) und dem Kopf (6) einen Hals (10) aufweist, wobei sich das Außengewinde (18) auf dem Hals (10) erstreckt.

4. Chirurgische Schraube nach Anspruch 1, **dadurch gekennzeichnet, dass** das Außengewinde (17) in das am Hals (10) angeordnete Außengewinde (18) übergeht, und/oder dass das am Hals (10) angeordnete Außengewinde (18) in das am Außenumfang (15) des Kopfes (6) angeordnete Außengewinde (16) übergeht.

5. Chirurgische Schraube nach einem oder mehreren der Ansprüche 1 bis 4, **dadurch gekennzeichnet, dass** Kenngrößen des Außengewindes (17) und des Außengewindes (18) und des Außengewindes (16) gleich sind.

6. Chirurgische Schraube nach Anspruch 4,
**dadurch gekennzeichnet,**
**dass** sich Kenngrößen des am Schaft (4) angeordneten Außengewindes (17) und des am Hals (10) angeordneten Außengewindes (18) und des am Kopf (6) angeordneten Außengewindes (16) unterscheiden.

7. Chirurgische Schraube nach einem der Ansprüche 5 oder 6, **dadurch gekennzeichnet, dass** die Kenngrößen des Außengewindes (16; 17; 18) ein Flankendurchmesser oder ein Kerndurchmesser oder ein Steigungswinkel des Außengewindes (16; 17; 18) oder die Mehrgängigkeit oder die Eingängigkeit des Außengewindes (16; 17; 18) sind.

8. Chirurgische Schraube nach einem oder mehreren der Ansprüche 1 bis 7, **dadurch gekennzeichnet, dass** der Kopf (6) am Schaft (4) der chirurgischen Schraube angelenkt ist.

9. Chirurgische Schraube nach einem oder mehreren der Ansprüche 1 bis 8, **dadurch gekennzeichnet, dass** der Kopf (6) eine zum Schaft (4) horizontal verlaufende Bohrung (24) aufweist, die zum Durchtritt des Stabilisierungselements ausgebildet ist.

10. Chirurgische Schraube nach Anspruch 9, **dadurch gekennzeichnet, dass** das Stabilisierungselement ein wenigstens zwei Knochen (1) oder wenigstens zwei Bereiche eines Knochens (1) miteinander verbindender Stab und/oder eine Platte oder ein Seil ist.

11. Chirurgische Schraube nach einem oder mehreren der Ansprüche 1 bis 10, **dadurch gekennzeichnet, dass** der Schaft (4) und der Hals (10) und/oder der Hals (10) und der Kopf (6) der chirurgischen Schraube jeweils konisch ineinander übergehen.

12. Chirurgische Schraube nach einem oder mehreren der Ansprüche 1 bis 11, **dadurch gekennzeichnet, dass** der Schaft (4) und der Hals (10) und der Kopf (6) der chirurgischen Schraube jeweils voneinander abweichende Durchmesser aufweisen.

13. Chirurgische Schraube nach einem oder mehreren der Ansprüche 1 bis 10, **dadurch gekennzeichnet, dass** der Schaft (4) und der Hals (10) und der Kopf (6) der chirurgischen Schraube gleiche Durchmesser aufweisen.

14. Chirurgische Schraube nach einem oder mehreren der Ansprüche 1 bis 13, **gekennzeichnet durch** einen axialen Kanal, der sich entlang der Längsachse (8) durch den Schaft (4) und/oder durch den Hals (10) und/oder durch den Kopf (6) erstreckt.

15. Chirurgische Schraube nach Anspruch 14, **dadurch gekennzeichnet, dass** zum Einführen wenigstens eines Gegenstands in den Kanal eine Zugeinrichtung angeordnet ist.

16. Chirurgische Schraube nach Anspruch 14, **dadurch gekennzeichnet, dass** in den Kanal ein Draht oder eine Innenschraube einführbar ist.

17. Chirurgische Schraube nach Anspruch 14, **dadurch gekennzeichnet, dass** zum Aufweiten des Schaftes (4) und/oder der Halses (10) und/oder des Kopfes (6) eine Spreizeinrichtung angeordnet ist.

## Claims

1. Surgical screw for fixing in a bone (1) of a human or animal body, having a head (6), having a shaft (4), and having an external thread (17) disposed on at least one segment (9) of the shaft (4) of the surgical screw, wherein the external thread (16) extends onto at least one segment (13) of the head (6) **characterized in that** the head (6) comprises a U-shaped recess formed by at least two outer walls (25) for receiving a stabilizing element.

2. Surgical screw according to claim 1, **characterized in that** the surgical screw is an intravertebral screw (3).

3. Surgical screw according to one of the claims 1 or 2 **characterized in that** the surgical screw comprises a neck (10) between the shaft (4) and the head (6), wherein the external thread (18) extends onto the neck (10).

4. Surgical screw according to claim 1, **characterized in that** the external thread (17) transitions into the external thread (18) disposed on the neck (10), and/or the external thread (18) disposed on the neck (10) transitions into the external thread (16) disposed on the circumference (15) of the head (6).

5. The surgical screw according to any one or more of the claims 1 through 4, **characterized in that** characteristic values of the external thread (17) and the external thread (18) and the external thread (16) are identical.

6. Surgical screw according to claim 4, **characterized in that** characteristic values of the external thread (17) disposed on the shaft (4) and the external thread (18) disposed on the neck (10) and the external thread (16) disposed on the head (6) are different from each other.

7. Surgical screw according to one of the claims 5 or 6, **characterized in that** the characteristic values of the external thread (16; 17; 18) are a flank diameter or a core diameter or a pitch angle of the external thread (16; 17; 18) or the multi-start or single-start nature of the external thread (16; 17; 18).

8. Surgical screw according to any one or more of the claims 1 through 7, **characterized in that** the head (6) is joined to the shaft (4) of the surgical screw by means of a hinge.

9. Surgical screw according to any one or more of the claims 1 through 8, **characterized in that** the head (6) comprises a hole (24) running horizontal to the shaft (4) and implemented for passing through the stabilizing element.

10. Surgical screw according to claim 9, **characterized in that** the stabilizing element is a bar and/or a plate or a cable connecting at least two bones (1) or at least two regions of a bone (1) to each other.

11. Surgical screw according to any one or more of the claims 1 through 10, **characterized in that** the shaft (4) and the neck (10) and/or the neck (10) and the head (6) of the surgical screw each transition conically into each other.

12. Surgical screw according to any one or more of the claims 1 through 11, **characterized in that** the shaft (4) and the neck (10) and the head (6) of the surgical screw each have a diameter deviating from each other.

13. Surgical screw according to any one or more of the claims 1 through 10, **characterized in that** the shaft (4) and the neck (10) and the head (6) of the surgical screw each have an identical diameter.

14. Surgical screw according to any one or more of the claims 1 through 13, **characterized by** an axial channel extending along the longitudinal axis (8) through the shaft (4) and/or through the neck (10) and/or through the head (6).

15. Surgical screw according to claim 14, **characterized in that** a tension device is disposed for introducing at least one object into the channel.

16. Surgical screw according to claim 14, **characterized in that** a wire or an internal screw can be introduced into the channel.

17. Surgical screw according to claim 14, **characterized in that** a spreading device is disposed for expanding the shaft (4) and/or the neck (10) and/or the head (6).

## Revendications

1. Vis chirurgicale destinée à être fixée dans un os (1) d'un corps humain ou animal, comprenant une tête (6), une tige (4) et un filetage extérieur (17) disposé sur au moins une partie (9) de la tige (4) de la vis chirurgicale, le filetage extérieur (16) s'étendant sur au moins une partie (13) de la tête (6), **caractérisée en ce que** la tête (6) présente un évidement en forme de U formé par au moins deux parois extérieures (25) pour recevoir un élément de stabilisation.

2. Vis chirurgicale selon la revendication 1, **caractérisée en ce que** la vis chirurgicale est une vis intravertébrale (3).

3. Vis chirurgicale selon l'une des revendications 1 ou 2, **caractérisée en ce que** la vis chirurgicale comporte un col (10) entre la tige (4) et la tête (6), le filetage extérieur (18) s'étendant sur le col (10).

4. Vis chirurgicale selon la revendication 1, **caractérisée en ce que** le filetage extérieur (17) se prolonge par le filetage extérieur (18) disposé sur le col (10), et/ou **en ce que** le filetage extérieur (18) disposé sur le col (10) se prolonge par le filetage extérieur (16) disposé sur la périphérie extérieure (15) de la tête (6).

5. Vis chirurgicale selon une ou plusieurs des revendications 1 à 4, **caractérisée en ce que** les caractéristiques du filetage extérieur (17) et du filetage extérieur (18) et du filetage extérieur (16) sont identiques.

6. Vis chirurgicale selon la revendication 4, **caractérisée en ce que** les caractéristiques du filetage extérieur (17) disposé sur la tige (4) et du filetage extérieur (18) disposé sur le col (10) et du filetage extérieur (16) disposé sur la tête (6) sont différentes.

7. Vis chirurgicale selon l'une des revendications 5 ou 6, **caractérisée en ce que** les caractéristiques du filetage extérieur (16 ; 17 ; 18) sont un diamètre de flanc ou un diamètre de noyau ou un angle d'inclination du filetage extérieur (16 ; 17 ; 18) ou le pas multiple ou le pas simple du filetage extérieur (16 ; 17 ; 18).

8. Vis chirurgicale selon une ou plusieurs des revendications 1 à 7, **caractérisée en ce que** la tête (6) est articulée sur la tige (4) de la vis chirurgicale.

9. Vis chirurgicale selon une ou plusieurs des revendications 1 à 8, **caractérisée en ce que** la tête (6) présente un alésage (24) s'étendant horizontalement par rapport à la tige (4) et configuré pour le passage de l'élément de stabilisation.

10. Vis chirurgicale selon la revendication 9, **caractérisée en ce que** l'élément de stabilisation est une tige et/ou une plaque ou un câble reliant entre eux au moins deux os (1) ou au moins deux zones d'un os (1).

11. Vis chirurgicale selon l'une ou plusieurs des revendications 1 à 10, **caractérisée en ce que** la tige (4) et le col (10) et/ou le col (10) et la tête (6) de la vis chirurgicale se rejoignent respectivement de manière conique.

12. Vis chirurgicale selon l'une ou plusieurs des revendications 1 à 11, **caractérisée en ce que** la tige (4) et le col (10) et la tête (6) de la vis chirurgicale présentent respectivement des diamètres différents les uns des autres.

13. Vis chirurgicale selon une ou plusieurs des revendications 1 à 10, **caractérisée en ce que** la tige (4) et le col (10) et la tête (6) de la vis chirurgicale présentent des diamètres identiques.

14. Vis chirurgicale selon une ou plusieurs des revendications 1 à 13, **caractérisée par** un canal axial s'étendant le long de l'axe longitudinal (8) à travers la tige (4) et/ou à travers le col (10) et/ou à travers la tête (6).

15. Vis chirurgicale selon la revendication 14, **caractérisée en ce qu'**un dispositif de traction est disposé pour introduire au moins un objet dans le canal.

16. Vis chirurgicale selon la revendication 14, **caractérisée en ce qu'**un fil ou une vis intérieure peut être introduit dans le canal.

17. Vis chirurgicale selon la revendication 14, **caractérisée en ce qu'**un dispositif d'écartement est disposé pour élargir la tige (4) et/ou le col (10) et/ou la tête (6).
